(19) Europäisches Patentamt
European Patent Office
Office européen des brevets

(11) Veröffentlichungsnummer: **0 189 473 B1**

(12) # EUROPÄISCHE PATENTSCHRIFT

(45) Veröffentlichungstag der Patentschrift: **27.05.92**

(51) Int. Cl.$^5$: **C07D 231/56**, C07D 235/06, C07D 235/08, C07D 235/28, C07D 249/18, C07D 285/12

(21) Anmeldenummer: **85904092.5**

(22) Anmeldetag: **14.08.85**

(86) Internationale Anmeldenummer: **PCT/DE85/00275**

(87) Internationale Veröffentlichungsnummer: **WO 86/01204 (27.02.86 86/05)**

Die Akte enthält technische Angaben, die nach dem Eingang der Anmeldung eingereicht wurden und die nicht in dieser Patentschrift enthalten sind.

(54) **NEUE DOPAMIN-DERIVATE, VERFAHREN ZU IHRER HERSTELLUNG UND IHRE VERWENDUNG ALS ARZNEIMITTEL.**

(30) Priorität: **15.08.84 DE 3430310**
**15.07.85 DE 3525563**

(43) Veröffentlichungstag der Anmeldung: **06.08.86 Patentblatt 86/32**

(45) Bekanntmachung des Hinweises auf die Patenterteilung: **27.05.92 Patentblatt 92/22**

(84) Benannte Vertragsstaaten: **AT BE CH DE FR GB IT LI LU NL SE**

(56) Entgegenhaltungen:
**DE-A- 2 026 402**
**DE-A- 2 813 523**
**US-A- 4 314 944**

**See also references of WO8601204**

(73) Patentinhaber: **SCHERING AKTIENGESELLSCHAFT Berlin und Bergkamen**
**Müllerstrasse 170/178 Postfach 65 03 11**
**W-1000 Berlin 65(DE)**

(72) Erfinder: **SCHÖLLKOPF, Klaus**
**Kurstr. 6**
**W-1000 Berlin 38(DE)**
Erfinder: **ALBRECHT, Rudolf**
**Wunsiedeler Weg 37**
**W-1000 Berlin 46(DE)**
Erfinder: **LEHMANN, Manfred**
**Lutherstr. 13**
**W-1000 Berlin 49(DE)**
Erfinder: **SCHRÖDER, Gertrud**
**Theodor-Francke-Str. 42**
**W-1000 Berlin 42(DE)**

EP 0 189 473 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft den Gegenstand der Patentansprüche. $C_{1-4}$ bzw. $C_{1-5}$-Alkyl in Formel I bedeuten niederes, gerad- oder verzweigtkettiges Alkyl mit bis zu fünf Kohlenstoffatomen, wie z.B. Methyl, Ethyl, i-Propyl, Pentyl, tert.-Butyl und 2-Methyl-butyl.

Für den Fall, daß Z in Rest A der Formel I die Hydroxygruppe bedeutet, ist diese Hydroxygruppe Bestandteil eines tautomeren Systems.

Das folgende tautomere System liegt vor, wobei beide tautomeren Grenzformen stabil sein können.

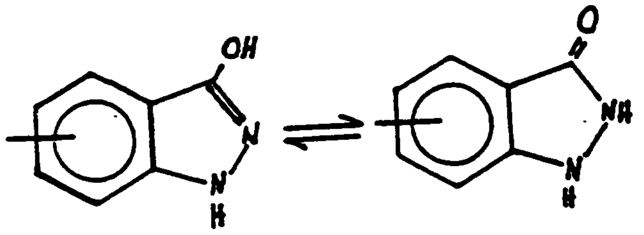

Bei allen Benzimidazol- und Benzotriazol-Derivaten sind zwei tautomere Formen in folgendem Sinne denkbar:

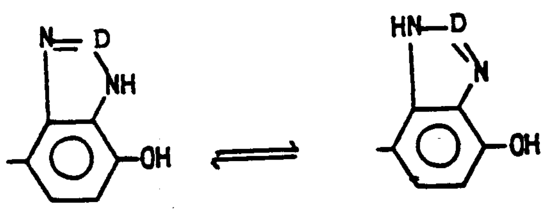

Zusätzlich besitzen Verbindungen mit $R^4$ = $NH_2$ und E =

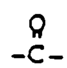 und 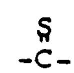

im Prinzip tautomeriefähige Strukturen, wobei folgende tautomere Grenzformen stabil sein können:

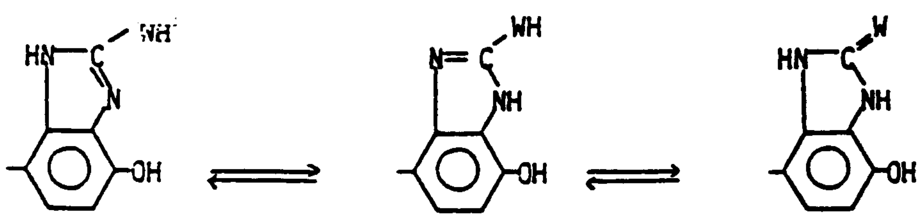 W = O, NH, S

Der natürliche Neurotransmitter Dopamin beeinflußt verschiedene biologische Prozesse, wie z. B. im Zentralnervensystem, wo dopaminerge Neurone an der Regulation der Motorkoordination und Prolactinausschüttung beteiligt sind. In der Peripherie verursacht Dopamin verschiedene cardiovaskauläre Effekte. So beeinflußt es die Gefäßspannung, ruft einen positiv inotropen und positiv chronotropen Effekt am Herzen hervor und fördert die Nierendurchblutung. Diese peripheren Effekte beruhen zum Teil auf dopaminergen, dh. durch Dopamin-Rezeptoren vermittelten Mechanismen, zum Teil auf anderen, z. B. adrenergen, Mechanismen. Wegen dieser mechanistischen Uneinheitlichkeit, abgesehen von der schlechten Bioverfügbarkeit nach oraler Applikation, ist Dopamin therapeutisch nur beschränkt verwendbar. Es wären daher Wirkstoffe wünschenswert, die auch peripher ganz oder zumindest überwiegend dopaminerge Wirkungsmechanismen aufweisen. So wurde z. B. das N,N-Dipropyldopamin entwickelt [Drugs of the Future 7, 469 (1982)], bei welchem die therapeutisch wichtige antihypertensive Wirkung hervortritt und welche fast ausschließlich über dopaminerge Mechanismen hervorgerufen wird. Ein Nachteil des N,N-Dipropyldopamins

ist jedoch, daß es nur eine sehr kurze Wirkungsdauer besitzt.

Des weiteren sind aus den US PS 3,574,741 und 3,758,692 Sulfonamidophenylalkylamine, wie das 5'-(2-Aminoethyl)-2'-hydroxy-methansulfonanilid, bekannt, das eine ausgeprägte vasokonstriktorische Wirkung zeigt . In DE-A-2026402 Werden Phenylethylaminderivate beschrieben, die zur Behandlüng von Migräne und venaler Hypertonie geeignet sind. Es finden sich Hinweise in der Literatur, wonach bereits versucht wurde, durch Aryläthylamin-Analoga mit Benzimidazol oder Benzotriazol als Arylteil zu Verbindungen mit therapeutisch nutzbaren Eigenschaften zu kommen.

So werden folgende Verbindungen beschrieben:

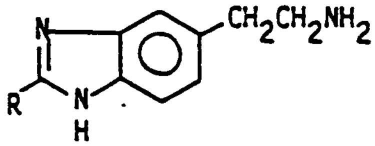

R = H, $CH_3$, 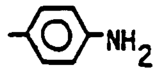

Diese Verbindungen besitzen jedoch blutdrucksteigernde Wirkung (DE-B- 294 085).

Desgleichen wurde eine ähnliche Verbindung mit einem Benzotriazol-Ringsystem hergestellt:

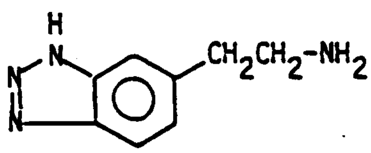 .

Nach Angaben der Hersteller besitzt diese Verbindung jedoch nicht die gewünschte dopaminerge Aktivität [H. Schmidhammer und K. Hohenlohe-Qehringen, Sci. Pharm. 51, 8 (1983)].

Es wurde nun gefunden, daß die Dopamin-Derivate der Formel I eine vasodilatatorische und antihypertensive Wirkung aufweisen, wobei die Wirkungsdauer im Vergleich zu N,N-Dipropyldopamin wesentlich verlängert ist.

Zur Ermittlung der biologischen Wirksamkeit der erfindungsgemäßen Verbindungen wurde die Beeinflussung des Blutdruckes in Abhängigkeit von der Zeit an wachen, spontan-hypertensiven Ratten, die nach der Methode von Weeks präpariert wurden, gemessen.

Dazu wurden die Substanzen in destilliertem Wasser oder physiologischer Kochsalzlösung gelöst und in die Vena jugularis injiziert. Die Blutdruckmessung erfolgte über einen chronisch implantierten Katheter in der Bauchaorta. Die Ergebnisse sind in der nachfolgenden Tabelle zusammengestellt. Die Meßwerte sind Mittelwerte von Versuchen an jeweils 3-6 Tieren.

Tabelle

| Blutdrucksenkung in Abhängigkeit von der Zeit | | | | |
|---|---|---|---|---|
| Verbindung | Dosis (mg/kg) | Blutdrucksenkung[4)] | | |
| | | Maximalwirkung (%) | nach 20 min[3)] (%) | nach 60 min[3)] (%) |
| N,N-Dipropyldopamin, Hydrobromid | 0.3[1)] | 15 | 0 | 0 |
| N,N-Dipropyl-N-[2-(7-amino-1H-indazol-4-yl)-ethyl]-amin, Dihydrochlorid | 10[2)] | 15 | 15 | 8 |

[1)]Infundiert während 20 Minuten

[2)]Bolus-Injection

[3)]Gemessen ab Ende der Applikation

[4)]Angegeben ist die Verminderung des Blutdruckes in % gegenüber dem Ausgangswert

Für das als Vergleichssubstanz gewählte N,N-Dipropyldopamin ist bekannt [I. Cavero et al., J. Pharmacol. Exp. Ther. 218, 515 (1981)], daß dessen Blutdruckbeeinflussung sehr schnell abklingt. So sind Messungen an verschiedenen Ratten-Modellen publiziert worden, denen zu entnehmen ist, daß je nach Modell und Versuchsdurchführung 6, 10, 15 oder 30 Minuten nach Beendigung der Substanzinjektion keine Wirkung mehr feststellbar ist. In dem oben genannten Modell der spontan-hypertensiven Ratte betrug die Blutdrucksenkung nach einer Gabe von 0,3 mg/kg N,N-Dipropyldopamin, Hydrobromid 5 Minuten nach Beendigung der Infusion bereits weniger als 1 % des Kontrollwertes, so daß zu den folgenden Meßzeiten, d. h. nach 20 und 60 Minuten, keine Wirkung der Substanz mehr feststellbar war.

In der zweiten Spalte der Tabelle ist die maximale Blutdrucksenkung in % der Verminderung gegenüber dem Ausgangswert aufgeführt, die mit den Verbindungen in der angegebenen Dosierung zu einem bestimmten Zeitpunkt während oder nach der Substanzinjektion erzielt wird. Daraus ist ersichtlich, daß eine vergleichbare Wirkung im Bereich von 15% Blutdrucksenkung für die erfindungsgemäße Verbindung im Vergleich zum N,N-Dipropyldopamin erst mit sehr viel höherer Dosierungen erzielt, wird die jedoch im Hinblick auf eine therapeutische Anwendung am Menschen tolerabel ist. Entscheidend ist jedoch, daß mit dieser äquieffektiven Dosis im Vergleich zu N,N-Dipropyldopamin eine sehr viel längere Wirkdauer erzielt wird, so daß für die erfindungsgemäße Verbindung auch noch 60 Minuten nach Applikationsende eine deutliche Blutdrucksenkung beobachtet wird.

Die erfindungsgemäßen Verbindungen sind somit als Antihypertensiva geeignet. Neben ihrer Anwendung als Antihypertensiva können sie aber auch zur Verbesserung der Nierendurchblutung und zum Auslösen eines diuretischen Effektes sowie in der Therapie zentralnervöser Störungen, wie z. B. bei Parkinson' scher Krankheit, Schizophrenie, Hyperprolactinämie, oder als Magentherapeutica, wie z. B. bei Hyperacidität oder Magenulcera, verwendet werden. Bei der Anwendung der erfindungsgemäßen Verbindung zur Erhöhung der Nierendurchblutung ist die gleichzeitige Gabe eines Diuretikums zur Erzielung eines Maximaleffektes möglich.

Zur Verwendung der erfindungsgemäßen Verbindungen als Arzneimittel werden diese nach an sich bekannten Methoden der Galenik in die Form eines pharmazeutischen Präparats gebracht, die neben dem Wirkstoff insbesondere für die enterale Applikation geeignete organische oder anorganische inerte Trägermaterialien, wie z. B. Wasser, Gelatine, Gummi arabicum, Milchzucker, Stärke, Magnesiumstearat, Talk, Polyalkylenglykole usw. enthalten können. Die pharmazeutischen Präparate können in fester Form, z. B. als Tabletten, Dragées, Suppositorien, Kapseln oder in flüssiger Form, z. B. als Lösungen, Suspensionen oder Emulsionen vorliegen. Gegebenenfalls enthalten sie darüber hinaus Hilfsstoffe wie Konservierungs-, Stabilisierungs-, Netzmittel oder Emulgatoren, Salze zur Veränderung des osmotischen Drucks oder Puffer.

Für die Verwendung am Menschen beträgt die Dosierung 50 bis 1000 mg pro Tag.

Die erfindungsgemäßen Verbindungen der Formel I werden nach an sich bekannten Methoden hergestellt.

Die Reduktion der Nitrogruppe in Verbindungen der Formel III gemäß Verfahrenvariante $a_1$) kann durch katalytisch angeregten Wasserstoff, oder mit Zink in Essigsäure, Eisen in Salzsäure, Zinn-II-chlorid usw. erfolgen.

Die Hydrolyse einer Aminogruppe gemäß Verfahrensvariante $a_2$) kann in Schwefelsäure bei erhöhter Temperatur durchgeführt werden.

Die Verbindungen der Formel I, worin A einen substituierten Phenylrest der Struktur

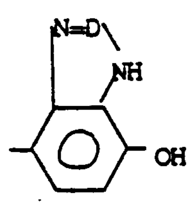

oder

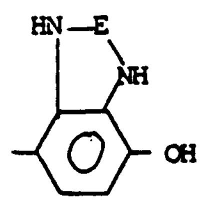

werden hergestellt, indem ein substituiertes Phenylethylamin der Formel IV

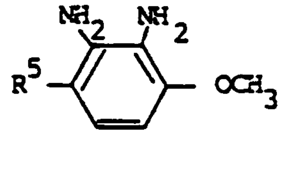

(IV),

worin $R^5$ Wasserstoff oder den Rest

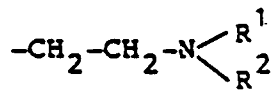

mit $R^1$ und $R^2$ in den bereits angegebenen Bedeutungen darstellt, mit einem Reagenz R

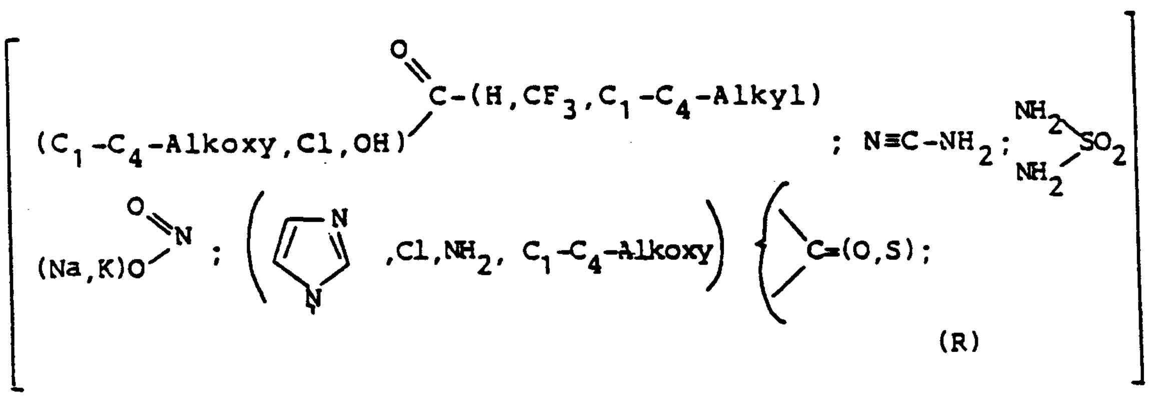

(R)

umsetzt und gegebenenfalls nach Einführung von $C_1$-$C_5$-Alkylgruppen am N-Atom der Aminoethyl-Seitenkette oder für den Fall, daß $R^5$ = H ist, nach Einführung einer Formylgruppe ($R^5$ = CHO), Umwandlung in eine Nitrovinylgruppe ($R^5 = CH = CH\text{-}NO_2$), Reduktion zu einer Aminoethylgruppe ($R^5 = CH_2\text{-}CH_2\text{-}NH_2$) und $C_1$-$C_5$-Alkylierung an dieser $CH_2\text{-}CH_2\text{-}NH_2$-Gruppe einer Etherspaltung unterwirft.

Die Darstellung der Ausgangsverbindungen IV und deren Umsetzung zu den Verbindungen der Formel I erfolgt nach an sich bekannten Methoden.

Die Umsetzung der Verbindungen der Formel IV mit dem entsprechenden Reagenz R als Carbonsäurederivat wird bevorzugt bei 100-120 ° C durchgeführt.

Die Umsetzung der Verbindungen der Formel IV mit Cyanamid wird in wäßrigen Mineralsäuren bei Temperaturen von 80-120 ° C durchgeführt.

Die Umsetzung von Verbindungen der Formel IV mit Alkalinitrat wird bevorzugt in wäßrigen Mineralsäuren oder wäßrigen Carbonsäuren durchgeführt. Beispielsweise wird eine Verbindung der Formel IV in 30%iger Essigsäure gelöst und mit Natriumnitrit bei Temperaturen von -5 bis +20 ° C umgesetzt.

Die Umsetzung von Verbindungen der Formel IV mit Kohlensäurederivaten wird bevorzugt in inerten Lösungsmitteln wie Toluol, niederen Alkoholen oder Tetrahydrofuran durchgeführt. Beispielsweise wird eine Verbindung der Formel IV in Tetrahydrofuran gelöst und mit Carbonyl-N,N'-diimidzol oder Thiocarbonyl-N,N'-diimidazol bei Temperaturen von 40-65 ° C umgesetzt.

Die Umsetzung von Verbindungen der Formel IV mit Sulfamid wird bevorzugt in einem hochsiedenden, inerten Lösungsmittel, wie z.B. Diglyme, bei Temperaturen von 80 ° C bis zur Siedetemperatur des Lösungsmittels durchgeführt.

Die Etherspaltung der so erhaltenen Verbindungen kann nach allen für diese Reaktion beschriebenen Methoden durchgeführt werden, erfolgt jedoch in zwei bevorzugten Ausführungsformen durch Erhitzen mit Bromwasserstoffsäure bei Temperaturan von 100-125 ° C oder mit Bortribromid in einem inerten Lösungsmittel wie Methylenchlorid bei Temperaturen von 0-40 ° C.

Die zur Herstellung der erfindungsgemäßen Verbindungen benötigten Ausgangsverbindungen sind entweder bekannt oder können wie folgt synthetisiert werden:

N,N-Dimethyl-N-[2-(7-nitro-1H-indazol-4yl)ethenyl]-amin

17,7 g 4-Methyl-7-nitro-1H-indazol [Chem. Ber. 37, 2556 (1904)] und 68,0 g tert.-Butoxy-bis-(dimethylamino)-methan werden 45 Minuten lang auf 80 ° C erwärmt, mit Diisopropylether versetzt, abgesaugt und mit Diisopropylether gewaschen. Man erhält 22,0 g N,N-Dimethyl-N-[2-(7-nitro-1H-indazol-4-yl)-ethenyl]-amin vom Schmelzpunkt 255 ° C.

N,N-Dimethyl-N- [2-(7-nitro-1H-indazol-4-yl)-ethyl]-amin

16,2 g N,N-Dimethyl-N-[2-(7-nitro-1H-indazol-4-yl)ethenyl]-aminwerden in 200 ml Essigsäure gelöst und unter Eiskühlung in 30 Minuten portionsweise mit 6,3 g Natriumcyanoborhydrid versetzt. Nach weiteren 30 Minuten versetzt man mit Eis und Essigsäureethylester, stellt mit konzentrierter Natronlauge pH 8-9 ein und extrahiert mit Essigsäureethylester. Die Lösung wird getrocknet, eingeengt und der Rückstand aus Essigsäureethylester/Diisopropylether umkristallisiert. Man erhält 13,8 g N,N-Dimethyl-N-[2-(7-nitro-1H-indazol-4-yl)-ethyl]-amin vom Schmelzpunkt 145-146° C.

N,N-Dipropyl-N-[2-(7-nitro-1H-indazol-4-yl)-ethenyl]-amin

2,1 g 4-Methyl-7-nitro-1H-indazol und 5,0 g tert.-Butoxy-bis-(dipropylamino)-methan werden in 10 ml Tetrahydrofuran 2,5 Stunden auf 80° C erwärmt. Man engt ein, destilliert mit Tetrahydrofuran nach und kristallisiert aus Dichlormethan/Hexan um, wobei 3,1 g N,N-Dipropyl-N-[2-(7-nitro-1H-indazol-4-yl)-ethenyl]-amin vom Schmelzpunkt 166-167° C erhalten werden.

N,N-Dipropyl-N-[2-(7-nitro-1H-indazol-4-yl)-ethyl]-amin. Hydrochlorid

2,9 g N,N-Dipropyl-N-[2-(7-nitro-1H-indazol-4-yl)-ethenyl]-aminwerden wie bei der Herstellung von N,N-Dimethyl-N-[2-(7-nitro-1H-indazol-4-yl)ethyl]-amin beschrieben reduziert. Das Produkt wird an Kieselgel mit Methanol/Dichlormethan 1:4 chromatographiert und mit etherischer Salzsäure in Methanol behandelt. Nach Umkristallisation aus Methanol/Diethylether werden 0,9 g N,N-Dipropyl-N-[2-(7-nitro-1H-indazol-4-yl)-ethyl]-amin, Hydrochlorid vom Schmelzpunkt 180,5 - 182,5° C erhalten.

2-(7-Nitro-1H-indazol-4-yl)acetonitril

23,3 g N,N-Dimethyl-N-[2-(7-nitro-1H-indazol-4-yl)ethenyl]-aminund 35,0 g Hydroxylamin-o-sulfonsäure in 400 ml Wasser werden 24 Stunden bei Raumtemperatur gerührt. Der Niederschlag wird aubgesaugt, mit Wasser gewaschen und aus Essigsäureethylester umkristallisiert, wobei 16,0 g 2-(7-nitro-1H-indazol-4yl)-acetonitril vom Schmelzpunkt 198 - 200° C erhalten werden.

2-Amino-3-methoxy-6-(2-trifluoracetamido-ethyl)-benzoesäure methylester und
2-Amino-3-methoxy-6-(2-trifluoracetamido-ethyl)-benzoesäure

2,84 g N-[2-(2,3-Dihydro-2,3-dioxo-7-methoxy-1H-indol-4-yl)-ethyl]-trifluoracetamid [J. Med. Chem. 26, 933 (1983)] werden in 40 ml Methanol, 10 ml Wasser und 10,8 ml ln Natronlauge gelöst, in 5 Minuten unter Eiskühlung mit 13 ml Wasserstoffperoxid (3 %) versetzt und 30 Minuten bei Raumtemperatur nachgerührt. Es wird mit 40 l Wasser versetzt und einmal mit Essigsäureethylester ausgeschüttelt (Fraktion 1).

Die wäßrige Phase wird mit in Salzsäure angesäuert und dreimal mit Essigsäureethylester ausgeschüttelt (Fraktion 2).

Fraktion 1 wird auf einer Kieselgel-Säule (System: Cyclohexan/Essigsäureethylester 1:1) gereinigt und aus Diisopropylether/Hexan umkristallisiert. Man erhält 1,0 g 2-Amino-3-methoxy-6-(2-trifluoracetamido-ethyl)-benzoesäuremethylester vom Schmelzpunkt 59 - 60° C. Nach Zugabe von etherischer Salzsäure wird das Hydrochlorid vom Schmelzpunkt 173 - 175° C (Zersetzung) erhalten.

Fraktion 2 wird auf einer Kieselgel-Säule (System: Dichlormethan/Methanol 4:1) gereinigt. Man erhält 1,3 g 2-Amino-3-methoxy-6- (2-trifluoracetamido-ethyl)-benzoesäure vom Schmelzpunkt 169 - 171° C (Zersetzung).

N-[2-(3-Hydroxy-7-methoxy-1H-indazol-4yl)-ethyl]-trifluoracetamid und
N-[2-(2.3-Dihydro-7-methoxy-3-oxo-1H-indazol-4-yl)-ethyl]-trifluoracetamid

6,6 g 2-Amino-3-methoxy-6-(2-trifluoracetamido-ethyl)-benzoesäuremethylester werden mit 30 ml 5n Salzsäure versetzt und bei einer Temperatur von - 3° C in 5 Minuten mit 1,5 g Natriumnitrit in 5 ml Wasser vereinigt. Man rührt 20 Minuten bei - 3° C nach, kühlt auf - 10° C ab und vereinigt mit einer Lösung von 12,9 g Zinn-II-chlorid, Dihydrat in 20 ml konzentrierter Salzsäure. Es wird 2,5 Stunden bei 0° C und 0,5 Stunden bei Raumtemperatur gerührt, der Niederschlag durch Versetzen mit Essigsäureethylester gelöst, erneut 0,5 Stunden bei Raumtemperatur gerührt, die organische Phase abgetrennt und die wäßrige Phase zweimal mit Essigsäureethylester gewaschen.

Die Essigsäureethylester-Lösungen werden eingeengt und der Rückstand mit gesättigter Natriumhydrogencarbonat-Lösung und Essigsäureethylester 16 Stunden bei Raumtemperatur gerührt. Nach

Einengen und Umkristallisieren aus Methanol/Diisopropylether werden 2,8 g N-[2-(3-Hydroxy-7-methoxy-1H-indazol-4-yl)-ethyl]-trifluoracetamid vom Zersetzungspunkt 213 - 217° C erhalten.

1,07 g der freien 2-Amino-3-methoxy-6-(2-trifluoracetamidoethyl)-benzoesäure werden wie vorstehend umgesetzt. Dabei werden 0,36 g N-[2-(3-Hydroxy-7-methoxy-1H-indazol-4-yl)-ethyl]-trifluoracetamid erhalten.

2-(3-Hydroxy-7-methoxy-1H-indazol-4-yl)-ethylamin, Hydrochlorid und
2-(2,3-Dihydro-7-methoxy-3-oxo-1H-indazol-4-yl)-ethylamin, Hydrochlorid

1,8 g N-[2-(3-Hydroxy-7-methoxy-1H-indazol-4-yl)-ethyl]-trifluoracetamid werden in 24 ml Ethanol, 6 ml Wasser und 6 ml konzentrierter Salzsäure 8 Stunden unter Rückfluß gekocht. Nach Einengen wird aus Methanol/Diethylether umkristallisiert, wobei 1,45 g 2-(3-Hydroxy-7-methoxy-1H-indazol-4-yl)-ethylamin, Hydrochlorid vom Zersetzungspunkt 230 - 232° C erhalten werden.

N,N-Dipropyl-N-[2-(3-hydroxy-7-methoxy-1H-indazol-4-yl)-ethyl]-amin, Hydrochlorid und
N,N-Dipropyl-N-[2-(2,3-dihydro-7-methoxy-3-oxo-1H-indazol-4-yl-ethyl]-amin, Hydrochlorid

1,45 g 2-(3-Hydroxy-7-methoxy-1H-indazol-4-yl)-ethylamin, Hydrochlorid in 40 ml Methanol werden mit 3,35 ml Propionaldehyd versetzt und anschließend unter Eiskühlung 688 mg Natriumcyanoborhydrid portionsweise zugefügt. Man rührt 3 Stunden bei 0° C und versetzt mit 20 ml In Salzsäure. Es wird 1 Stunde bei Raumtemperatur nachgerührt, im Vakuum eingeengt, die Lösung mit Natriumhydrogencarbonat alkalisch eingestellt und viermal mit Essigsäureethylester ausgeschüttelt. Die vereinigten Essigsäureethylester-Phasen werden getrocknet, eingeengt und über eine Kieselgel-Säule chromatographiert (System: Toluol/Eisessig/Wasser). Das Produkt wird in Methanol mit etherischer Salzsäure behandelt, wobei 700 mg N,N-Dipropyl-N-[2-(3-hydroxy-7-methoxy-1H-indazol-4-yl)-ethyl]-amin, Hydrochlorid vom Schmelzpunkt 198 - 201° C erhalten werden.

2,3-Dihydro-3-hydroxy-7-methoxy-2-oxo-5-(2-trifluoracetamidoethyl)-3-indol-carbonsäureethylester

2,6 g N-[2-(4-Amino-3-methoxyphenyl)-ethyl]-trifluoracetamid und 1,9 ml Mesoxalsäurediethylester werden in 25 ml Essigsäure gelöst und 4 Stunden unter Rückfluß gekocht. Man engt ein, löst den Rückstand in Essigsäureethylester und schüttelt zweimal mit gesättigter Natriumhydrogencarbonat-Lösung aus. Es wird eingeengt und an Kieselgel mit Cyclohexan/Essigsäureethylester 1:1 chromatographiert. Man erhält 2,5 g 2,3-Dihydro-3-hydroxy-7-methoxy-2-oxo-5-(2-trifluoracetamidoethyl)-3-indol-carbonsäureethylester vom Schmelzpunkt 115 - 117° C.

2-Amino-3-methoxy-5-(2-trifluoracetamidoethyl)-benzoesaüremethylester

3,9 g 2,3-Dihydro-3-hydroxy-7-methoxy-2-oxo-5-(2-trifluoracetamidoethyl)-3-indol-carbonsäureeethylester werden in 100 ml Methanol und 20 ml In Natronlauge gelöst und 5 Stunden lang Luft durchgeleitet. Es wird mit 25 ml In Salzsäure versetzt und mit Essigsäureethylester ausgeschüttelt. Das Produkt wird über Kieselgel mit Cyclohexan/Essigester 1:1 chromatographiert, wobei nach Umkristallisation aus Ethanol/Wasser 2,1 g 2-Amino-3-methoxy-5-(2-trifluoracetamidoethyl)-benzoesäuremethylester vom Schmelzpunkt 86 - 87° C erhalten werden.

N-[2-(3-Hydroxy-7-methoxy-1H-indazol-5-yl)-ethyl]-trifluoracetamid und
N-[2-(2,3-Dihydro-7-methoxy-3-oxo-1H-indazol-5-yl)-ethyl]-trifluoracetamid

2,3 g 2-Amino-3-methoxy-5-(2-trifluoracetamidoethyl)-benzoesäuremethylester werden in 10 ml 5n Salzsäure suspendiert und wie bei der Herstellung von N-[2-(3-Hydroxy-7-methoxy-1H-indazol-4-yl)-ethyl]-trifluoracetamid beschrieben umgesetzt.

Man erhält 950 mg N-[2-(3-Hydroxy-7-methoxy-1H-indazol-5-yl)-ethyl]-trifluoracetamid vom Zersetzungspunkt 232-233° C.

2-(3-Hydroxy-7-methoxy-1H-indazol-5-yl)-ethylamin, Hydrochlorid und
2-(2,3-Dihydro-7-methoxy-3-oxo-1H-indazol-5-yl)-ethylamin, Hydrochlorid

2,7 g N-[2-(3-Hydroxy-7-methoxy-1H-indazol-5-yl)-ethyl]-trifluoracetamid werden wie bei der Herstellung

von 2-(3-Hydroxy-7-methoxy-1H-indazol-4-yl)-ethylamin, Hydrochlorid mit Salzsäure gekocht, wobei durch Kristallisation aus Ethanol/Diethylether 1,5 g 2-(3-Hydroxy-7-methoxy-1H-indazol-5-yl)-ethylamin, Hydrochlorid vom Zersetzungspunkt 266 - 268° C erhalten werden.

N,N-Dipropyl-N-[2-(3-hydroxy-7-methoxy-1H-indazol-5-yl)-ethyl]-amin, Hydrochlorid und
N-[2-(2,3)-Dihydro-7-methoxy-3-oxo-1H-indazol-5-yl)-ethyl]-N,N-dipropylamin, Hydrochlorid

2-(3-Hydroxy-7-methoxy-1H-indazol-5-yl)-ethylamin, Hydrochlorid wird wie bei der Herstellung von N,N-Dipropyl-N-[2-(3-hydroxy-7-methoxy-1H-indazol-4-yl)-ethyl]-amin, Hydrochlorid beschrieben mit Propionaldehyd umgesetzt, wobei N,N-Dipropyl-N-[2-(3-hydroxy-7-methoxy-1H-indazol-5-yl)-ethyl]-amin, Hydrochlorid erhalten wird.

N,N-Dimethyl-N-[2-(7-nitro-1H-indazol-5-yl)-ethenyl]-amin

5-Methyl-7-nitroindazol [Chem. Ber. 29, 306 (1896)]wird wie bei der Herstellung von N,N-Dimethyl-N-[2-(7-nitro-1H-indazol-4-yl)-ethenyl]-amin beschrieben mit tert.-Butoxy-bis-(dimethylamino)-methan umgesetzt, wobei N,N-Dimethyl-N-[2-(7-nitro-1H-indazol-5-yl)-ethenyl]-amin erhalten wird.

N,N-Dimethyl-N-[2-(7-nitro-1H-indazol-5-yl)-ethyl]-amin

N,N-Dimethyl-N-[2-(7-nitro-1H-indazol-5-yl)-ethenyl]-amin wird wie bei der Herstellung von N,N-Dimethyl-N-[2-(7-nitro-1H-indazol-4-yl)-ethyl]-amin beschrieben mit Natriumcyanborhydrid umgesetzt, wobei N,N-Dimethyl-N-[2-(7-nitro-1H-indazol-5-yl)-ethyl]-amin entsteht.

Bis-dipropylamino-tert.-butoxy-methan

a) Zu 92.1 g Dimethylsulfat werden 95 g N,N-Dipropylformamid zugetropft. Anschließend wird für 3 Stunden auf 75 °C erhitzt. Nach dem Abkühlen wird mit Diethylether extrahiert und der Rückstand wird bei 55 °C im Wasserstrahlvakuum getrocknet. Es werden 185 g N,N-Dipropylammonio-ameisensäuremethylester Methylsulfat als Öl erhalten.
b) 127.5 g Dipropylamin werden in 450 ml Toluol gelöst. Dazu werden 185 g der unter a) erhaltenen Verbindung zugetropft. Nach 48 Stunden bei Raumtemperatur wird für 2.5 Stunden auf 85 °C erhitzt. Nach dem Erkalten werden die Phasen getrennt, das Produkt wird mit Diethylether extrahiert und anschließend bei 55 °C im Wasserstrahlvakuum getrocknet. Es werden 230 g N,N,N',N'-Tetrapropyl-formamidinium-methylsulfat als Öl erhalten.
c) 230 g der unter b) erhaltenen Verbindung werden mit 500 ml Diethylether gut durchgerührt. Dazu werden 85 g Kaliumtertiärbutylat portionsweise zugegeben. Anschließend wird das Gemisch für 2 Stunden am Rückfluß erhitzt. Nach dem Abkühlen wird abfiltriert und der Rückstand mit Diethylether nachgewaschen. Nach dem Abdestillieren des Lösungsmittels wird der Rückstand im Vakuum destilliert. Es werden 26 g Bis-dipropylamino-tert.-butoxy-methan vom Siedepunkt 125 °C bei 0.03 mbar erhalten.

N-[2-(2,3-Diamino-4-methoxyphenyl)-ethyl]-N,N-dimethylamin

a) 57.7 g 4-Methoxy-2,3-dinitrotoluol (J. Chem. Soc. 1927, 580) werden mit 150 ml Bis-dimethylamino-tert.-butoxy-methan [Chem. Ber. 101, 41 (1968)] für 6 Stunden bei 80 °C gerührt. Nach dem Abkühlen wird der ausgefallene Feststoff abfiltriert, mit Diisopropylether nachgewaschen und im Wasserstrahlvakuum getrocknet. Es werden 54.6 g β-Dimethylamino-4-methoxy-2,3-dinitrostyrol vom Schmelzpunkt 100-131 °C erhalten.
b) 25 g der unter a) erhaltenen Verbindung werden in einem Gemisch aus 250 ml Methanol, 180 ml Tetrahydrofuran und 37 ml Eisessig gelöst und auf -20 °C abgekühlt. Zu dieser Lösung werden 6.3 g Natriumcyanborhydrid portionsweise zugegeben. Anschließend wird noch 2 Stunden bei 0 °C nachgerührt. Nach erneutem Abkühlen auf -20 °C werden 140 ml 2-normale Salzsäure zugegeben. Anschließend wird 1 Stunde bei 0 °C nachgerührt. Das Reaktionsgemisch wird eingeengt und der Niederschlag wird abfiltriert und aus Methanol umkristallisiert. Es werden 21.2 g N-[2-(4-Methoxy-2,3-dinitrophenyl)-ethyl]-N,N-dimethylamin Hydrochlorid vom Schmelzpunkt 223-225 °C erhalten.
c) 5.7 g der unter b) erhaltenen Verbindung werden mit Natriumhydrogencarbonat-Lösung in die freie Base überführt. Diese wird in Essigester aufgenommen und nach Zugabe von 4 g Raney-Nickel bei Raumtemperatur und Normaldruck hydriert. Nach beendeter Wasserstoffaufnahme wird der Katalysator

abfiltriert und das Filtrat eingeengt. Es werden 3.2 g N-[2-(2,3-Diamino-4-methoxyphenyl)-ethyl]-N,N-dimethylamin als Öl erhalten.

N-[2-(2,3-Diamino-4-methoxyphenyl)-ethyl]-N,N-dipropylamin

a) 5.0 g 4-Methoxy-2,3-dinitrotoluol werden mit 7.5 g Bis-dipropylamino-tert.-butoxy-methan für eine Stunde auf 60 °C erhitzt. Nach dem Abkühlen wird das Reaktionsgemisch im Hochvakuum von flüchtigen Bestandteilen befreit. Es werden 7.1 g β-Dipropylamino-4-methoxy-2,3-dinitrostyrol als Öl erhalten.
b) 6.2 g der unter a) erhaltenen Verbindung werden in einem Gemisch aus 54 ml Methanol, 30 ml Tetrahydrofuran und 7.5 ml Eisessig gelöst und auf -20 °C abgekühlt. Zu dieser Lösung werden 0.8 g Natriumcyanborhydrid portionsweise zugegeben. Anschließend wird 2 Stunden bei 20 °C nachgerührt. Zu der Lösung werden dann 30 ml 2-normale Salzsäure gegeben und es wird eine Stunde nachgerührt. Das Reaktionsgemisch wird daraufhin eingeengt und in Natriumhydrogencarbonatlösung aufgenommen. Die wäßrige Phase wird mehrmals mit Diethylether extrahiert. Die Etherphase wird abgetrennt, mit Natriumsulfat getrocknet und mit Oxalsäure versetzt. Der Niederschlag wird abfiltriert und mit Diethylether nachgewaschen. Anschließend wird mit Natriumhydrogencarbonatlösung die Base freigesetzt und mit Diethylether extrahiert. Nach Entfernen des Lösungsmittels erhält man 1.2 g N-[2-(4-Methoxy-2,3-dinitrophenyl)-ethyl]-N,N-dipropylamin als Öl.
c) 0.7 g der unter b) erhaltenen Verbindung werden in 100 ml Essigester gelöst und nach Zugabe von 1 g Raney-Nickel hydriert. Nach beendeter Wasserstoffaufnahme wird der Katalysator abfiltriert und das Filtrat eingeengt. Es werden 0.53 g N-[2-(2,3-Diamino-4-methoxyphenyl)-ethyl]-N,N-dipropylamin als Öl erhalten.

Die nachfolgenden Beispiele sollen das erfindungsgemäße Verfahren erläutern.

Beispiel 1

N,N-Dimethyl-N-[2-(7-amino-1H-indazol-4-yl)-ethyl]amin, Dihydrochlorid

1,2 g N,N-Dimethyl-N-[2-(7-nitro-1H-indazol-4-yl)-ethyl]-amin in 60 ml Tetrahydrofuran werden in Gegenwart von 1,0 g Raney-Nickel hydriert. Man filtriert, engt ein und kristallisiert aus Essigsäureethylester/Hexan um. Man erhält 860 mg N,N-Dimethyl-N-[2-(7-amino-1H-indazol-4-yl)ethyl]-amin vom Schmelzpunkt 125° C. 460 mg dieses Produktes werden mit Ethanol und etherischer Salzsäure behandelt, wobei nach Umkristallisation aus Methanol/Diethylether 505 mg N,N-Dimethyl-N-[2-(7-amino-1H-indazol-4-yl)-ethyl]-amin, Dihydrochlorid, vom Schmelzpunkt 231 - 232° C erhalten werden.

Beispiel 2

N,N-Dimethyl-N-[2-(7-hydroxy-1H-indazol-4-yl)-ethyl]-amin, Hydrochlorid

1,2 g N,N-Dimethyl-N-[2-(7-amino-1H-indazol-4-yl)-ethyl]-amin und 24 ml In Schwefelsäure werden 16 Stunden unter Argon im Autoklav auf 180° C erwärmt. Anschließend wird unter Eiskühlung mit Natriumhydrogencarbonat versetzt und mit Essigsäureethylester/Butanol (10:1) ausgeschüttelt. Die organische Phase wird getrocknet, eineengt und der Rückstand mit Essigsäureethylester verrieben. Man erhält 700 mg N,N-Dimethyl-N-[2-(7-hydroxy-1H-indazol-4-yl)-ethyl]-amin vom Schmelzpunkt 233 - 234° C, welches nach Behandeln mit Methanol und etherischer Salzsäure und Umkristallisieren aus Methanol/Diethylether 680 mg N,N-Dimethyl-N-[2-(7-hydroxy-1H-indazol-4-yl)-ethyl]-amin, Hydrochlorid vom Schmelzpunkt 223 - 227° C liefert.

Beispiel 3

N-[4-(2-Dimethylaminoethyl)-1H-indazol-7-yl]-formamid, Hydrochlorid

10 ml Ameisensäure und 0,9 ,l Essigsäureanhydrid werden 15 Minuten unter Rückfluß gekocht und bei Raumtemperatur mit 1,2 g N,N-Dimethyl-N-[2-(7-amino-1H-indazol-4-yl)-ethyl]-amin versetzt. Nach 16 Stunden bei Raumtemperatur engt man ein, versetzt mit gesättigter Natriumhydrogencarbonatlösung und extrahiert dreimal mit Butanol/Essigsäureethylester (1: 5). Das Produkt wird an Kieselgel mit Methanol/Dichlormethan 1:1 chromatographiert, mit etherischer Salzsäure versetzt und aus

Methanol/Diethylether umkristallisiert, wobei 950 mg N-[4-(2-Dimethylaminoethyl)-1H-indazol-7-yl]-formamid, Hydrochlorid vom Schmelzpunkt 210-211° C erhalten werden.

Beispiel 4

N-[4-(2-Dimethylaminoethyl)-1H-indazol-7-yl]-acetamid, Hydrochlorid

306 mg N,N-Dimethyl-N-[2-(7-amino-1H-indazol-4-yl-ethyl]-amin in 4 ml Essigsäure werden 16 Stunden mit 0,18 ml Essigsäureanhydrid gerührt. Das Produkt wird an Kieselgel mit Methanol/Dichlormethan 1:1 chromatographiert und mit etherischer Salzsäure ins Hydrochlorid überführt. Man erhält 180 mg N-[4-(2-Dimethylaminoethyl)-1H-indazol-7-yl]-acetamid, Hydrochlorid vom Schmelzpunkt 223° C (aus Methanol/Diethylether).

Beispiel 5

N-[4-(2-Dimethylaminoethyl)-1H-indazol-7-yl]-methansulfonamid, Hydrochlorid

1,2 g N,N-Dimethyl-N-[2-(7-amino-1H-indazol-4-yl)-ethyl]-amin in 10 ml Dimethylformamid und 5 ml Tetrahydrofuran werden bei 0° C mit 0,91 ml Methansulfonsäurechlorid versetzt. Man rührt 2 Stunden bei 0° C und 14 Stunden bei Raumtemperatur. Das Produkt wird an Kieselgel mit Methanol/Dichlormethan 1:1 chromatographiert und mit etherischer Salzsäure behandelt, wobei nach Umkristallisation aus Methanol/Diethylether 550 mg N-[4-(2-Dimethylaminoethyl)-1H-indazol-7-yl]-methansulfonamid,Hydrochlorid vom Schmelzpunkt 203 - 205° C erhalten werden.

Beispiel 6

N,N-Dipropyl-N-[2-(7-amino-1H-indazol-4-yl)-ethyl]-amin, Dihydrochlorid

600 mg N,N-Dipropyl-N-2-(7-nitro-1H-indazol-4-yl)-ethylamin, Hydrochlorid in 10 ml Methanol und 10 ml Tetrahydrofuran werden in Gegenwart von 0,5 g Raney-Nickel hydriert. Man filtriert vom Katalysator ab, versetzt mit etherischer Salzsäure und engt ein. Der Rückstand wird aus Methanol/Diethylether umkristallisiert. Man erhält 360 mg N,N-Dipropyl-N-2-(7-amino-1H-indazol-4-yl)-ethylamin, Dihydrochlorid vom Schmelzpunkt 214 - 218° C.

Beispiel 7

N,N-Dipropyl-N-[2-(7-hydroxy-1H-indazol-4-yl)-ethyl]-amin, Dihydrochlorid

a) 20,2 g 2-(7-Nitro-1H-indazol-4-yl)-acetonitril in 750 ml Tetrahydrofuran werden in Gegenwart von 20,0 g Raney-Nickel hydriert. Das Produkt wird aus Tetrahydrofuran/Hexan umkristallisiert, wobei 15,1 g 2-(7-Amino-1H-indazol-4-yl)-acetonitril vom Schmelzpunkt 164 - 165° C erhalten werden.

b) 3,44 g des unter a) beschriebenen Produktes und 80 ml ln Schwefelsäure werden 20 Stunden unter Argon im Autoklaven auf 170° C erwärmt. Man saugt das Kristallisat ab und wäscht mit Wasser, wobei 3,4 g 2-(7-Hydroxy-1H-indazol-4-yl)-essigsäure vom Schmelzpunkt 258-260° C (Zersetzung) erhalten werden.

c) 2,3 g des unter b) beschriebenen Produktes in 10 ml Dimethylformamid und 15 ml Tetrahydrofuran werden bei - 10° C mit 1,56 ml N-Ethylmorpholin und 1,68 ml Chlorameisenisobutylester versetzt, 5 Minuten danach werden 2,46 ml Dipropylamin in 20 ml Tetrahydrofuran zugesetzt. Man rührt 0,5 Stunden bei 0° C und 16 Stunden bei Raumtemperatur, engt ein und nimmt mit Essigsäureethylester und Wasser auf. Aus der Essigsäureethylesterlösung werden nach Umkristallisation aus dem gleichen Lösungsmittel 2,1 g 2-(7-Hydroxy-1H-indazol-4-yl)-essigsäure-dipropylamid vom Schmelzpunkt 164 - 165° C erhalten.

d) 1,66 g 2-(7-Hydroxy-1H-indazol-4-yl)-essigsäure-dipropylamid in 150 ml Tetrahydrofuran werden unter Eiskühlung mit 1,5 g Lithiumaluminiumhydrid versetzt und 5 Stunden bei Raumtemperatur gerührt. Unter Eiskühlung wird vorsichtig mit Wasser versetzt und Essigsäureethylester/gesättigte Natriumhydrogencarbonat-Lösung zugegeben. Das aus der Essigsäureethylesterlösung erhaltene Produkt wird an Kieselgel mit Toluol/Essigsäure/Wasser 10:10:1 chromatographiert und mit etherischer Salzsäure in Ethanol behandelt, wobei 1,8 g N,N-Dipropyl-N-[2-(7-hydroxy-1H-indazol-4-yl)-ethyl]-amin,

Dihydrochlorid vom Schmelzpunkt 125 - 127° C erhalten werden.

Beispiel 8

N,N-Diallyl-N-[2-(7-hydroxy-1H-indazol-4-yl)-ethyl]-amin, Dihydrochlorid

a) 2-(7-Hydroxy-1H-indazol-4-yl)-essigsäure wird wie im Beispiel 19 c) beschrieben mit Diallylamin umgesetzt, wobei 2-(7-Hydroxy-1H-indazol-4-yl)-essigsäurediallyamid vom Schmelzpunkt 107 - 108° C erhalten wird.
b) Das unter a) erhaltene Produkt wird wie im Beispiel 7 d) beschrieben mit Lithiumaluminiumhydrid umgesetzt, wobei N, N-Diallyl-N[2-(7-hydroxy-1H-indazol-4-yl)-ethyl]-amin, Hydrochlorid vom Schmelzpunkt 213° C erhalten wird.

Beispiel 9

N-[2-(7-Hydroxy-1H-indazol-4-yl)-ethyl]-amin, Hydrochlorid

a) 2,3 g 2-(7-Hydroxy-1H-indazol-4-yl)-essigsäure werden analog Beispiel 7 c) mit Benzylamin anstelle von Dipropylamin umgesetzt. Das Produkt wird aus Methanol/Diisopropylether umkristallisiert, wobei 2,6 g 2-(7-Hydroxy-1H-indazol-4-yl)-essigsäurebenzylamid vom Schmelzpunkt 188 - 190° C erhalten werden.
b) 2,2 g 2-(7-Hydroxy-1H-indazol-4-yl)-essigsäure-benzylamid in 60 ml Tetrahydrofuran werden unter Argon in 1,9 g Lithiumaluminiumhydrid in 50 ml Tetrahydrofuran eingetroft und 16 Stunden unter Rückfluß gekocht. Anschließend wird wie im Beispiel 7 d) beschrieben aufgearbeitet. Das Produkt wird an Kieselgel mit Methanol/Chloroform 1:1 chromatographiert, mit etherischer Salzsäure behandelt und aus Methanol/Diethylether umkristallisiert, wobei 1,2 g N-Benzyl-N-[2-(7-hydroxy-1H-indazol-4-yl)-ethyl]-amin, Hydrochlorid vom Schmelzpunkt 163 - 165° C (Zersetzung) erhalten werden.
c) 870 mg N-Benzyl-N-[2-(7-hydroxy-1H-indazol-4-yl)-ethyl]-amin, Hydrochlorid in 50 ml Methanol werden in Gegenwart von 1,0 g Palladium-Kohle hydriert. Das Produkt wird aus Ethanol/Diethylether umkristallisiert, wobei 565 mg N-[2-(7-Hydroxy-1H-indazol-4-yl)-ethyl]-amin, Hydrochlorid vom Schmelzpunkt 257 - 260° C erhalten werden.

Beispiel 10

2-(7-Amino-1H-indazol-4-yl)-ethylamin, Dihydrochlorid

344 mg 2-(7-Amino-1H-indazol-4-yl)-acetonitril in 10ml Essigsäureanhydrid werden bei 1000 kPa in Gegenwart von 0,5 g Raney-Nickel bei Raumtemperatur hydriert. Das Reaktionsprodukt wird an Kieselgel mit Methanol/Dichlormethan chromatographiert. Das isolierte N-[2-(7-Acetamino-1H-indazol-4-yl)-ethyl]-acetamid (340 mg, Schmelzpunkt 211 - 212° C) wird in 2 ml Essigsäure und 5 ml Salzsäure 5 Stunden unter Rückfluß gekocht, eingeengt und der Rückstand aus Ethanol umkristallisiert, wobei 220 mg 2-(7-Amino-1H-indazol-4-yl)-ethylamin, Dihydrochlorid vom Schmelzpunkt 288-291° C (Zersetzung) erhalten werden.

Beispiel 11

N-[4-(2-Aminoethyl)-1H-indazol-7-yl]-methansulfonamid, Hydrochlorid

a) 1,7 g 2-(7-Amino-1H-indazol-4-yl)-acetonitril in 15 ml Dimethylformamid und 5 ml Tetrahydrofuran werden bei 0° C mit 0,96 ml Methansulfonsäurechlorid und 1,56 ml N-Ethylmorpholin versetzt und 16 Stunden bei Raumtemperatur gerührt. Man engt ein, chromatographiert über Kieselgel mit Methanol/Dichlormethan (1:3) und kristallisiert aus Methanol um, wobei 2,2 g N-(4-Cyanomethyl-1H-indazol-7-yl)-methansulfonamid vom Schmelzpunkt 223 - 225° C erhalten werden.
b) 1,4 g N-(4-Cyanomethyl-1H-indazol-7-yl)-methansulfonamid in 60 ml ethanolischer Ammoniak-Lösung werden in Gegenwart von 2,0 g Raney-Nickel bei 80° C und 8000 kPa in 6 Stunden hydriert. Man filtriert vom Katalysator ab, engt ein, chromatographiert über eine Kieselgel-Säule (Eluens: Methanol/Dichlormethan 1:1), versetzt mit etherischer Salzsäure und kristallisiert aus Methanol/Diethylether um. Man erhält 500 mg N-[4-(2-Aminoethyl)-1H-indazol-7-yl]-methansulfonamid, Hydrochlorid vom Schmelzpunkt 120° C (Zersetzung).

Beispiel 12

N-[2-(3,7-Dihydroxy-1H-indazol-4-yl)-ethyl]-N,N-dipropylamin, Hydrobromid und
N-[2-(2,3-Dihydro-7-hydroxy-3-oxo-1H-indazol-4-yl)-ethyl]-N,N-dipropyl-amin, Hydrobromid

480 mg N,N-Dipropyl-N-[2-(3-hydroxy-7-methoxy-1H-indazol-4-yl)-ethyl]-amin, Hydrochlorid werden in 5 ml wäßrigem Bromwasserstoff (65 %) 4 Stunden unter Argon auf 120° C erwärmt. Man engt ein und destilliert einmal mit Wasser nach. Der Rückstand wird getrocknet, mit Diethylether verrieben und mehrmals aus Ethanol/Aceton/Diethylether umkristallisiert. Man erhält 340 mg N-[2-(3,7-Dihydroxy-1H-indazol-4-yl-)-ethyl]-N,N-dipropyl-amin, Hydrobromid vom Zersetzungs punkt 242 - 244° C.

Beispiel 13

2-(3,7-Dihydroxy-1H-indazol-4-yl)-ethylamin, Hydrobromid und
2-(2,3-Dihydro-7-hydroxy-3-oxo-1H-indazol-4-yl)-ethylamin, Hydrobromid

750 mg N-[2-(3-Hydroxy-7-methoxy-1H-indazol-4-yl]-trifluoracetamid werden mit 5 ml wäßrigem Bromwasserstoff (65 %) 2 Stunden unter Argon auf 120° C erwärmt. Das Kristallisat wird aus Methanol/Diethylether umkristallisiert, wobei 680 mg 2-(3,7-Dihydroxy-1H-indazol-4-yl)-ethylamin, Hydrobromid vom Zersetsungspunkt 270 - 273° C erhalten werden.

Beispiel 14

2-(3,7-Dihydroxy-1H-indazol-5-yl)-ethylamin, Hydrobromid und
2-(2,3-Dihydro-7-hydroxy-3-oxo-1H-indazol-5-yl)-ethylamin, Hydrobromid

450 mg N-[2-(3-Hydroxy-7-methoxy-1H-indazol-5-yl)-ethyl]-trifluoracetamid in 10 ml wäßrigem Bromwasserstoff (65 %) werden 3 Stunden unter Argon auf 120° C erwärmt. Der Niederschlag wird aus Methanol/Diethylether umkristallisiert, wobei 400 mg 2-(2,7-Dihydroxy-1H-indazol-5-yl)-ethylamin, Hydrobromid vom Zersetzungspunkt 253 - 255° C erhalten werden.

Beispiel 15

N-[2-(3,7-Dihydroxy-1H-indazol-5-yl)-ethyl]-N,N-dipropylamin, Hydrobromid und
N-[2-(2,3-Dihydro-7-hydroxy-3-oxo-1H-indazol-5-yl)-ethyl]-N,N-dipropyl-amin, Hydrobromid

N,N-Dipropyl-N-[2-(3-hydroxy-7-methoxy-1H-indazol-5-yl)-ethyl]-amin, Hydrochlorid wird wie im Beispiel 12 beschrieben mit Bromwasserstoff umgesetzt, wobei N-[2-(3,7-Dihydroxy-1H-indazol-5-yl)-ethyl]-N,N-dipropyl-amin, Hydrobromid erhalten wird.

Beispiel 16

N-[2-(7-Amino-1H-indazol-5-yl)-ethyl]-N,N-dimethyl-amin, Dihydrochlorid

N,N-Dimethyl-N-[2-(7-nitro-1H-indazol-5-yl)-ethyl]-amin wird wie im Beispiel 13 beschrieben hydriert und mit Salzsäure behandelt, wobei N-[2-(7-Amino-1H-indazol-5-yl)-ethyl]-N,N-dimethyl-amin, Dihydrochlorid entsteht.

Beispiel 17

N,N-Dimethyl-N-[2-(7-hydroxy-1H-indazol-5-yl)-ethyl]-amin, Hydrochlorid

N-[2-(7-Amino-1H-indazol-5-yl)-ethyl]-N,N-dimethyl-amin, Dihydrochlorid wird wie im Beispiel 2 beschrieben mit Schwefelsäure hydrolysiert, wobei N,N-Dimethyl-N-[2-(7-hydroxy-1H-indazol-5-yl)-ethyl]-amin, Hydrochlorid entsteht.

BEISPIEL 18

4-[2-(N,N-Dimethylamino)-ethyl]-benzimidazol-7-ol, Dihydrobromid

a) 0.30 g N-[2-(2,3-Diamino-4-methoxyphenyl)-ethyl]-N,N-dimethylamin werden mit 10 ml Ameisensäure 3 Stunden am Rückfluß erhitzt. Die Lösung wird filtriert und eingeengt, wobei 0.23 g 7-Methoxy-4-[2-(N,N-dimethylamino)-ethyl]-benzimidazol, Formiat als glasige Masse erhalten werden.
b) 225 mg der unter a) erhaltenen Verbindung werden mit 5 ml 48-prozentiger Bromwasserstoffsäure 3 Stunden am Rückfluß erhitzt. Nach dem Einengen wird der Rückstand aus einem Ethanol/Diethylether-Gemisch umkristallisiert. Es werden 260 mg 4-[2-(N,N-Dimethylamino)-ethyl]-benzimidazol-7-ol, Dihydrobromid vom Schmelzpunkt 245-252 °C erhalten.

BEISPIEL 19

4-[2-(N,N-Dipropylamino)-ethyl]-benzimidazol-7-ol, Dihydrobromid

a) 1.2 g N-[2-(2,3-Diamino-4-methoxyphenyl)-ethyl]-N,N-dipropylamin werden mit 20 ml Ameisensäure 3 Stunden am Rückfluß erhitzt. Die Lösung wird eingeengt. Der Rückstand wird in Wasser aufgenommen, mit Kaliumcarbonat alkalisch gestellt und mit Essigester extrahiert. Die organische Phase wird über Natriumsulfat getrocknet und nach Abfiltrieren des Trockenmittels eingeengt. Der Rückstand wird an Kieselgel mit einem Gemisch aus Dichlormethan/Methanol chromatographiert. Es werden 0.42 g 7-Methoxy-4-[2-(N,N-dipropylamino)-ethyl]-benzimidazolals Öl erhalten.
b) 0.42 g der unter a) erhaltenen Verbindung werden in 10 ml Dichlormethan gelöst. Zu der auf 0 °C gekühlten Lösung werden 2.0 g Bortribromid zugetropft. Das Reaktionsgemisch wird langsam auf Raumtemperatur erwärmt. Nach 14 Stunden wird erneut auf 0 °C abgekühlt und es werden 10 ml Methanol zugegeben. Das Gemisch wird eingeengt und der Rückstand wird aus Isopropanol/Diethylether umkristallisiert. Es werden 0.44 g 4-[2-(N,N-dipropylamino)-ethyl]-benzimidazol-7-ol, Dihydrobromid vom Schmelzpunkt 135-139 °C erhalten.

BEISPIEL 20

4-[2-(N,N-Dimethylamino)-ethyl]-2-methyl-benzimidazol-7-ol, Dihydrobromid

a) 0.6 g N-[2-(2,3-Diamino-4-methoxyphenyl)-ethyl]-N,N-dimethylamin werden mit 10 ml Essigsäure 3 Stunden am Rückfluß erhitzt. Das Reaktionsgemisch wird eingeengt, in Essigester aufgenommen und mit Natriumhydrogencarbonatlösung ausgeschüttelt. Die organische Phase wird abgetrennt, mit Natriumsulfat getrocknet und eingeengt. Es werden 286 mg 7-Methoxy-4-[2-(N,N-dimethylamino)-ethyl]-2-methyl-benzimidazol als Öl erhalten.
b) 225 mg der unter a) erhaltenen Verbindung werden mit 5 ml 48-prozentiger Bromwasserstoffsäure 3 Stunden am Rückfluß erhitzt. Nach dem Einengen wird der Rückstand aus einem Isopropanol/Diethylether-Gemisch umkristallisiert. Es werden 178 mg 4-[2-(N,N-Dimethylamino)-ethyl]-2-methyl-benzimidazol-7-ol, Dihydrobromid mit einem Schmelzpunkt größer als 280 °C erhalten.

BEISPIEL 21

2-Butyl-4-[2-(N,N-dimethylamino)-ethyl]-benzimidazol-7-ol, Dihydrobromid

a) 500 mg N-[2-(2,3-Diamino-4-methoxyphenyl)-ethyl]-N,N-dimethylamin werden mit 10 ml Valeriansäure 7 Stunden auf 120 °C erhitzt. Das Reaktionsgemisch wird eingeengt. Der Rückstand wird in Diethylether aufgenommen und mit 4-normaler Salzsäure extrahiert. Die wäßrige Phase wird mit Kaliumhydrogencarbonatlösung neutralisiert und mit Essigester extrahiert. Die organische Phase wird mit Natriumsulfat getrocknet und eingeengt. Der Rückstand wird aus Essigester umkristallisiert. Es werden 170 mg 2-Butyl-7-methoxy-4-[2-(N,N-dimethylamino)-ethyl]-benzimidazol vom Schmelzpunkt 116-122 °C erhalten.
b) 130 mg der unter a) erhaltenen Verbindung werden mit 3 ml 48-prozentiger Bromwasserstoffsäure 4 Stunden am Rückfluß erhitzt. Nach dem Einengen wird der Rückstand aus einem Isopropanol/Diethylether-Gemisch umkristallisiert. Es werden 108 mg 2-Butyl-4-[2-(N,N-dimethylamino)-ethyl]-benzimidazol-7-ol, Dihydrobromid vom Schmelzpunkt 97-105 °C erhalten.

BEISPIEL 22

4-[2-(N,N-Dimethylamino)-ethyl]-2-trifluormethyl-benzimidazol-7-ol, Hydrobromid

a) 600 mg N-[-2-(2,3-Diamino-4-methoxyphenyl)-ethyl]-N,N-dimethylamin werden mit 10 ml Trifluoressigsäure 3 Stunden am Rückfluß erhitzt. Nach dem Einengen wird der Rückstand aus Isopropanol umkristallisiert. Es werden 908 mg 7-Methoxy-4-[2-(N,N-dimethylamino)-ethyl]-2-trifluormethyl-benzimidazol, Trifluoracetat vom Schmelzpunkt 182-185 °C erhalten.
b) 400 mg der unter a) erhaltenen Verbindung werden mit 3 ml 48-prozentiger Bromwasserstoffsäure 3 Stunden am Rückfluß erhitzt. Nach dem Einengen wird der Rückstand aus Butanol umkristallisiert. Es werden 100 mg 4-[2-(N,N-Dimethylamino)ethyl]-2-trifluormethyl-benzimidazol-7-ol, Hydrobromid vom Schmelzpunkt 198-200 °C erhalten.

BEISPIEL 23

7-Hydroxy-4-[2-(N,N-dimethylamino)-ethyl]-2,3-dihydro-2-benzimidazolon, Hydrobromid

a) 500 mg N-[2-(2,3-Diamino-4-methoxyphenyl)-ethyl]-N,N-dimethylamin werden mit 40 ml Tetrahydrofuran und 500 mg N,N'-Carbonyldiimidazol 3 Stunden am Rückfluß erhitzt. Nach dem Einengen wird der Rückstand mit Methanol und Wasser versetzt. Es wird erneut eingeengt und über Phosphorpentoxid getrocknet. Es werden 410 mg 7-Methoxy-4-[2-(N,N-dimethylamino)-ethyl]-2,3-dihydro-2-benzimidazolon vom Schmelzpunkt 197-199 °C erhalten.
b) 200 mg der unter a) erhaltenen Verbindung werden mit 10 ml 48-prozentiger Bromwasserstoffsäure 2 Stunden am Rückfluß erhitzt. Nach dem Einengen wird der Rückstand aus einem Isopropanol/Methanol/Diethylether-Gemisch umkristallisiert. Es werden 180 mg 7-Hydroxy-4-[2-(N,N-dimethylamino)-ethyl]-2,3-dihydro-2-benzimidazolon, Hydrobromid vom Schmelzpunkt 263-269 °C erhalten.

BEISPIEL 24

7-Hydroxy-4-[2-(N,N-dipropylamino)-ethyl]-2,3-dihydro-2-benzimidazolon, Hydrobromid

a) 530 mg N-[2-(2,3-Diamino-4-methoxyphenyl)-ethyl]-N,N-dipropylamin werden mit 30 ml Tetrahydrofuran und 500 mg N,N'-Carbonyldiimidazol eine Stunde am Rückfluß erhitzt. Nach dem Einengen wird der Rückstand mit Methanol und Wasser versetzt. Es wird erneut eingeengt und über Phosphorpentoxid getrocknet. Es werden 530 mg 7-Methoxy-4-[2-(N,N-dipropylamino)-ethyl]-2,3-dihydro-2-benzimidazolon vom Schmelzpunkt 35 °C erhalten.
b) 200 mg der unter a) erhaltenen Verbindung werden mit 10 ml 48-prozentiger Bromwasserstoffsäure 4 Stunden am Rückfluß erhitzt. Nach dem Einengen wird der Rückstand aus einem Isopropanol/Diethylether-Gemisch umkristallisiert. Es werden 170 mg 7-Hydroxy-4-[2-(N,N-dipropylamino)-ethyl]-2,3-dihydro-2-benzimidazolon, Hydrobromid vom Schmelzpunkt 250-255 °C erhalten.

BEISPIEL 25

2-Amino-4-[2-(N,N-dimethylamino)-ethyl]-benzimidazol-7-ol, Dihydrobromid

a) 1.0 g N-[2-(2,3-Diamino-4-methoxyphenyl)-ethyl]-N,N-dimethylamin werden mit 0.4 ml konzentrierter Salzsäure, 217 mg Cyanamid und 0.5 ml Wasser 5 Stunden am Rückfluß erhitzt. Anschließend werden 0.5 ml einer 50-prozentigen Kalilauge zugegeben und erneut für 4 Stunden am Rückfluß erhitzt. Anschließend wird eingeengt und der Rückstand wird mit Isopropanol extrahiert. Nach Zugabe von Bromwasserstoffsäure wird der entstehende Niederschlag aus Ethanol umkristallisiert. Es werden 260 mg 2-Amino-7-methoxy-4-[2-(N,N-dimethylamino)-ethyl]-benzimidazol, Dihydrobromid mit einem Zersetzungspunkt von 260 °C erhalten.
b) 260 mg der unter a) erhaltenen Verbindung werden mit 5 ml 63-prozentiger Bromwasserstoffsäure 2 Stunden am Rückfluß erhitzt. Nach dem Einengen wird der Rückstand aus Ethanol umkristallisiert. Es werden 91 mg 2-Amino-4-[2-(N,N-dimethylamino)-ethyl]-benzimidazol-7-ol, Dihydrobromid mit einem Zersetzungspunkt von 250 °C erhalten.

BEISPIEL 26

4-[2-(N,N-Dimethylamino)-ethyl]-benzotriazol-7-ol, Dihydrobromid

a) 1.0 g N-[2-(2,3-Diamino-4-methylphenyl)-ethyl]-N,N-dimethylamin werden in 1.2 ml Essigsäure und 2.4 ml Wasser gelöst. Dazu wird eine Lösung von 425 mg Natriumnitrit in 0.7 ml Wasser getropft. Nach 20 Minuten wird mit 2-normaler Ammoniaklösung neutralisiert und eingeengt. Der Rückstand wird mit Bromwasserstoffsäure versetzt und aus Methanol umkristallisiert. Es werden 1.0 g 7-Methoxy-4-[2-(N,N-dimethylamino)-ethyl]-benzotriazol,Hydrobromid vom Schmelzpunkt 176-180 °C erhalten.

b) 1.0 g der unter a) erhaltenen Verbindung werden mit 20 ml 48-prozentiger Bromwasserstoffsäure 5 Stunden am Rückfluß erhitzt. Nach dem Einengen wird der Rückstand aus Isopropanol umkristallisiert. Es werden 220 mg 4-[2-(N,N-Dimethylamino)-ethyl]-benzotriazol-7-ol, Dihydrobromid vom Schmelzpunkt 207-211 °C erhalten.

BEISPIEL 27

7-Hydroxy-4-[2-(N,N-dimethylamino)-ethyl]-2,3-dihydro-2-benzimidazolthion, Hydrobromid

a) 500 mg N-[2-(2,3-Diamino-4-methoxyphenyl)-ethyl]-N,N-dimethylamin werden mit 50 ml Tetrahydrofuran und 430 mg N,N'-Thiocarbonyldimidazol 3 Stunden am Rückfluß erhitzt. Nach dem Einengen wird der Rückstand mit Wasser versetzt. Der Niederschlag wird aus Ethanol umkristallisiert. Es werden 470 mg 7-Methoxy-4-[2-(N,N-dimethylamino)-ethyl]-2,3-dihydro-2-benzimidazolthion vom Schmelzpunkt 213-216 °C erhalten.

b) 200 mg der unter a) erhaltenen Verbindung werden mit 5 ml 63-prozentiger Bromwasserstoffsäure 2 Stunden am Rückfluß erhitzt. Nach dem Einengen wird der Rückstand aus einem Isopropanol/Diethylether-Gemisch umkristallisiert. Es werden 85 mg 7-Hydroxy-4-[2-(N,N-dimethylamino)-ethyl]-2,3-dihydro-2-benzimidazolthion, Hydrobromid mit einem Zersetzungspunkt von 210 °C erhalten.

BEISPIEL 28

7-Hydroxy-4-(2-aminoethyl)-2,3-dihydro-2-benzimidazolon, Hydrobromid

a) 1.9 g 4-Methoxy-2,3-dihydro-2-benzimidazolon (Dt. Offenlegungsschrift 28 19 458 (16.11.78)) werden mit 40 ml Methylenchlorid, 6.0 g Zinntetrachlorid und 2.7 g Dichlormethylmethylether im Ultraschallbad 4 Stunden lang gehalten (Eigenerwärmung). Es wird weitgehend eingeengt und der Rückstand mit Wasser versetzt. Nach dem Abfiltrieren und Trocknen erhält man 1.8 g 7-Methoxy-2,3-dihydro-2-benzimidazolon-4-aldehyd vom Schmelzpunkt 256-262 °C.

b) 1.8 g der unter a) erhaltenen Verbindung werden mit 0.7 g Nitromethan, 50 ml Methanol und 0.6 g Natriumhydroxid in 10 ml Wasser umgesetzt. Nach 30 Minuten wird auf eiskalte, halbkonzentrierte Salzsäure gegeben und der Niederschlag abfiltriert. Nach dem Umkristallisieren aus Ethanol werden 1.2 g 7-Methoxy-4-(2-nitrovinyl)-2,3-dihydro-2-benzimidazolon mit einem Zersetzungspunkt von 250 °C erhalten.

c) 1.2 g der unter b) erhaltenen Verbindung werden mit 100 ml Methanol, 100 ml Wasser, 10 ml 48-prozentiger Bromwasserstoffsäure und 400 mg Palladium-Aktivkohle versetzt und bei 50 °C hydriert. Nach beendeter Wasserstoffaufnahme wird vom Katalysator abfiltriert und nach dem Einengen werden 1.2 g 7-Methoxy-4-(2-aminoethyl)-2,3-dihydro-2-benzimidazolon, Hydrobromid vom Schmelzpunkt 180-190 °C erhalten.

d) Die Spaltung der Methoxyverbindung wird analog Beispiel 6b) ausgeführt, wobei 7-Hydroxy-4-(2-aminoethyl)-2,3-dihydro2-benzimidazolon, Hydrobromid erhalten wird.

BEISPIEL 29

7-Hydroxy-4-[2-(N-butyl-N-propylamino)-ethyl]-2,3-dihydro-2-benzimidazolon, Hydrobromid

Die Verbindung nach Beispiel 11c) wurde mit Propionylchlorid acyliert, anschließend mit Boran in Tetrahydrofuran reduziert, das Produkt mit Butyrylchlorid erneut acyliert und mit Boran in Tetrahydrofuran reduziert. Die entstandene Verbindung wird anschließend mit Bromwasserstoffsäure der Etherspaltung unterworfen, wobei 7-Hydroxy-4-[2-(N-butyl-N-propylamino)-ethyl]-2,3-dihydro-2-benzimidazolon, Hydrobromid erhalten wird.

BEISPIEL 30

7-Hydroxy-4-[2-(N,N-dimethylamino)-ethyl]-2,1,3-benzothiadiazol-2,2-dioxid, Hydrobromid

a) 2.0 g N-[2-(2,3-Diamino-4-methoxyphenyl)-ethyl]-N,N-dimethylamin werden in 17 ml Diglyme gelöst und am Rückfluß erhitzt. Zur siedenden Mischung wird eine Lösung von 1.12 g Sulfamid in 5 ml Diglyme gegeben. Nach 10 Minuten wird schnell abgekühlt. Der Niederschlag wird abfiltriert und aus Ethanol umkristallisiert. Es werden 1.12 g 7-Methoxy-4-[2-(N,N-dimethylamino)-ethyl]-2,1,3-benzothiadiazol-2,2-dioxid vom Schmelzpunkt 202-207 °C erhalten.

b) 500 mg der unter a) erhaltenen Verbindung werden mit 20 ml einer 1-molaren Bortribromidlösung in Dichlormethan gemischt. Nach 48 Stunden wird Methanol zugegeben und das Reaktionsgemisch zur Trockne eingeengt. Der Rückstand wird aus Ethanol umkristallisiert. Es werden 332 mg 7-Hydroxy-4-[2-(N,N-dimethylamino)-ethyl]-2,1,3-benzothiadiazol-2,2-dioxid, Hydrobromid vom Schmelzpunkt 202-205 °C erhalten.

**Patentansprüche**

**1.** Dopamin-Derivate der allgemeinen Formel I

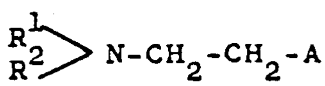

(I),

worin

A einen substituierten Phenylrest der Struktur

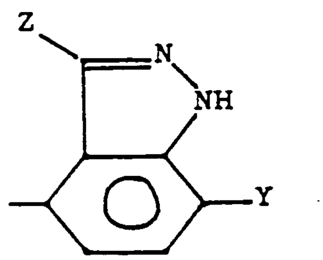

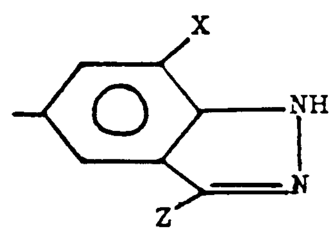

,

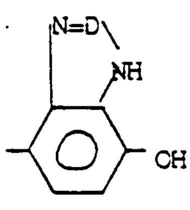

oder

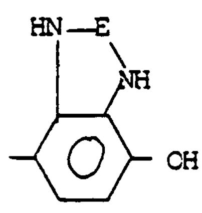

darstellt, worin

$R^1$ und $R^2$ gleich oder verschieden, Wasserstoff, $C_{1-5}$-Alkyl und Allyl,
D gleich

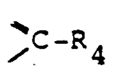

oder

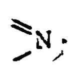

$R_4$ gleich Wasserstoff, $C_1$-$C_4$-Alkyl, $CF_3$ oder $NH_2$,

E gleich

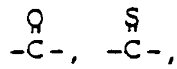

$-SO_2-$,
X = OH, $NH_2$,

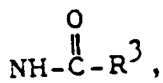

und $NH-SO_2-CF_3$,
Y = OH, $NH_2$,

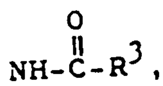

$NH-SO_2-CF_3$ und
$NH-SO_2-CH_3$,
mit $R^3$ = $C_{1-4}$-Alkyl
und
Z = H oder OH bedeuten und falls Z die Hydroxygruppe bedeutet, der Rest A auch in der tautomeren Grundform vorliegen kann,
und deren Säure-Additionssalze,

**2.** 7-Hydroxy-4-[2-(N,N-di-propylamino)-ethyl]-2,3-dihydro-2-benzimidazolon, Hydrobromid.

**3.** N,N-Dipropyl-N-[2-(7-amino-1H-indazol-4-yl)-ethyl]-amin und dessen Dihydrochlorid.

**4.** Verfahren zur Herstellung von Dopamin-Derivaten der Formel I

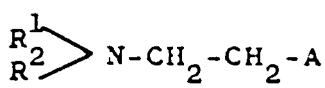 (I),

worin

A einen substituierten Phenylrest der Struktur

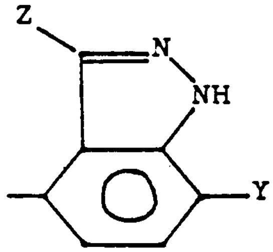

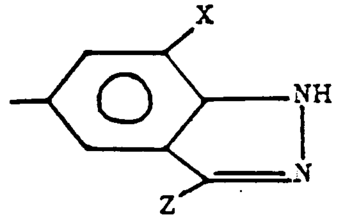

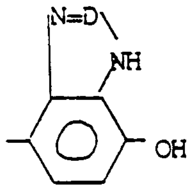

oder

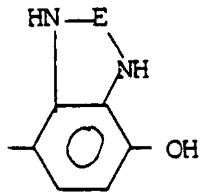

darstellt, worin

$R^1$ und $R^2$ gleich oder verschieden, Wasserstoff, $C_{1-5}$-Alkyl und Allyl,
D

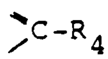 oder 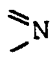

$R_4$ Wasserstoff, $C_1$-$C_4$-Alkyl, $CF_3$, $NH_2$
E

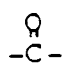 , 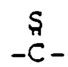 ,

$-SO_2-$,
X = OH, $NH_2$,

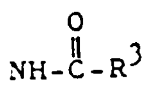 ,

und $NH-SO_2-CF_3$,
Y = OH, $NH_2$,

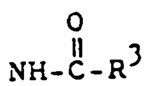 ,

$NH-SO_2-CF_3$ und
$NH-SO_2-CH_3$,
mit $R^3$ = $C_{1-4}$-Alkyl
und

Z = H oder OH bedeuten, und deren Säuren-Additionssalzen

dadurch gekennzeichnet, daß man

a) eine Verbindung der Formel III

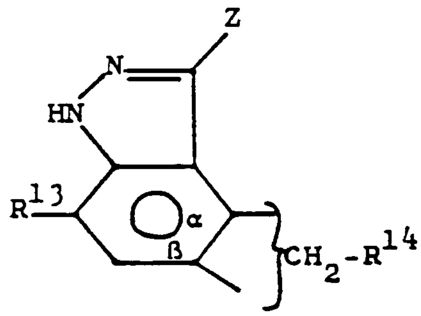

(III),

worin Z die oben angegebene Bedeutung hat, $R^{14}$ für $CH_2$-$NR^1R^2$ ($R^1$ und $R^2$ haben die oben angegebene Bedeutung), CN oder $CH_2$-NH-$COCF_3$ steht und $R^{13}$ gleich $NO_2$, $NH_2$ oder $OCH_3$ ist, wobei die Gruppe $CH_2$-$R^{14}$ entweder in $\alpha$- oder $\beta$-Stellung sich befinden kann,

a1) falls $R^{13}$ gleich $NO_2$ ist, zur Aminogruppe reduziert,

a2) falls $R^{13}$ gleich $NH_2$ ist, zur Hydroxygruppe hydrolysiert, oder

a3) die Aminogruppe mit einem Carbonsäurechlorid $R^3COCl$, Carbonsäureanhydrid $(R^3\text{-}CO)_2O$, wobei $R^3$ die oben angegebene Bedeutung hat, oder mit Methansulfosäurechlorid umsetzt, wobei Verbindungen der Formel I erhalten werden, in denen X die Bedeutung $R^3CONH$ oder $CH_3SO_2NH$ hat,

a4) falls $R^{13}$ gleich $OCH_3$ ist, einer Etherspaltung unterwirft, wobei Verbindungen der Formel I erhalten werden, bei denen X die Hydroxygruppe bedeutet, und falls $R^{14}$ die Gruppierung $CH_2$-NH-$COCF_3$ darstellt, $R^1$ und $R^2$ Wasserstoff sind.

b) ein substituiertes Phenylethylamin der Formel IV

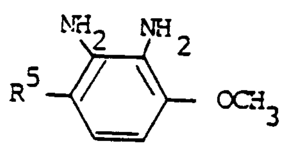

(IV),

worin $R^5$ Wasserstoff oder den Rest

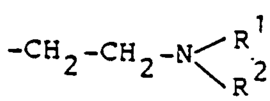

mit $R^1$ und $R^2$ in den bereits angegebenen Bedeutungen darstellt, mit einem Reagenz R

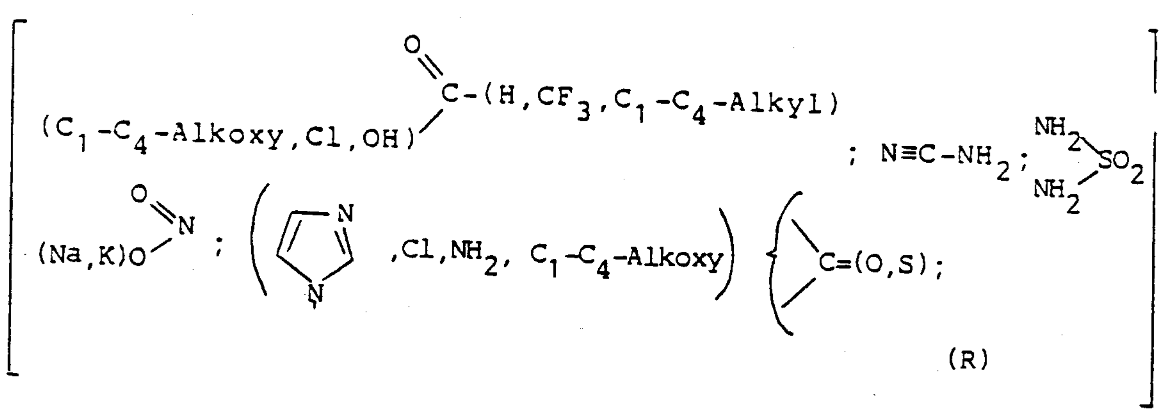

(R)

umsetzt und gegebenenfalls nach Einführung von $C_1$-$C_5$-Alkylgruppen am N-Atom der Aminoethyl-Seitenkette oder für den Fall, daß $R^5$ = H ist, nach Einführung einer Formylgruppe ($R^5$ = CHO), Umwandlung in eine Nitrovinylgruppe ($R^5$ = $CH=CH-NO_2$), Reduktion zu einer Aminoethylgruppe ($R^5$ = $CH_2-CH_2-NH_2$) und $C_1$-$C_5$-Alkylierung an dieser $CH_2-CH_2-NH_2$-Gruppe einer Etherspaltung unterwirft.

**5.** Arzneimittel, bestehend aus einer oder mehreren Verbindungen gemäß Anspruch 1 und üblichen Hilfs- und Trägerstoffen.

**Claims**

**1.** Dopamine derivatives of the general formula I

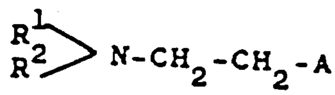

(I)

wherein

A represents a substituted phenyl radical of the structure

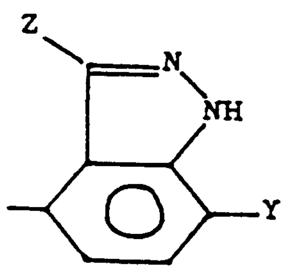

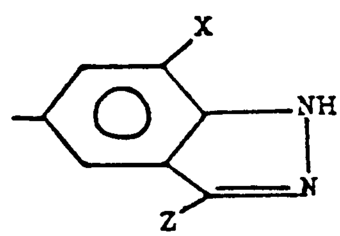

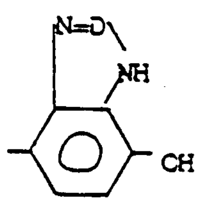

or

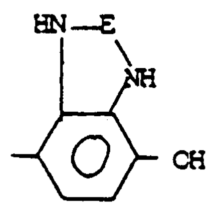

wherein

$R^1$ and $R^2$, being identical or different, are hydrogen, $C_{1-5}$-alkyl or allyl,

D is

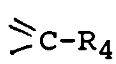

or

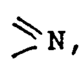

$R_4$ is hydrogen, $C_1$-$C_4$-alkyl, $CF_3$ or $NH_2$,

E is

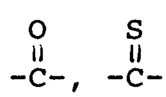

or $-SO_2-$,

X is OH, $NH_2$,

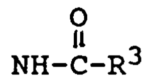

or $NH-SO_2-CF_3$,
Y is OH, $NH_2$,

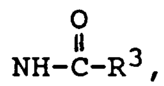

$NH-SO_2-CF_3$ or $NH-SO_2-CH_3$, with $R^3$ being $C_{1-4}$-alkyl,
and
Z is H or OH, and,
if Z represents the hydroxy group, the radical A can also be in the tautomeric base form,
and their acid addition salts.

**2.** 7-Hydroxy-4-[2-(N,N-dipropylamino)ethyl]-2,3-dihydro-2-benzimidazolone, hydrobromide.

**3.** N,N-Dipropyl-N-[2-(7-amino-1H-indazol-4-yl)ethyl]-amine and its dihydrochloride.

**4.** Process for the preparation of dopamine derivatives of formula I

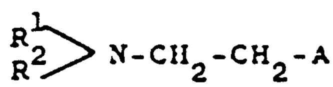

(I)

wherein
A represents a substituted phenyl radical of the struc

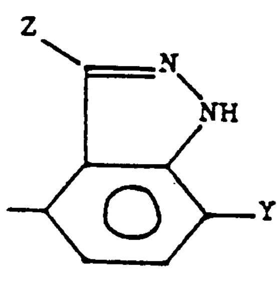

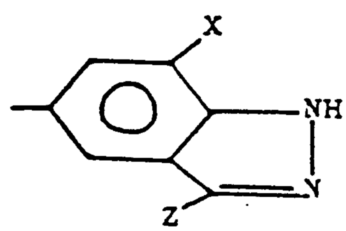

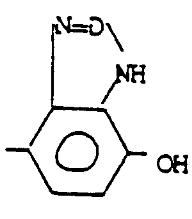

or

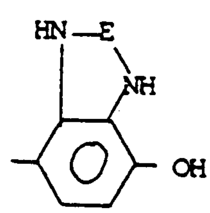

ture wherein
$R^1$ and $R^2$, being identical or different, are hydrogen, $C_{1-5}$-alkyl or allyl,
D is

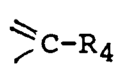 or 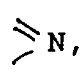

$R_4$ is hydrogen, $C_1$-$C_4$-alkyl, $CF_3$ or $NH_2$,
E is

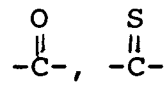

or $-SO_2-$,
X is OH, $NH_2$,

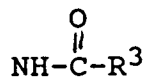

or $NH-SO_2-CF_3$,
Y is OH, $NH_2$,

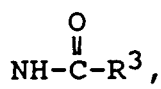

$NH-SO_2-CF_3$ or $NH-SO_2-CH_3$, with $R^3$ being $C_{1-4}$-alkyl,
and
Z is H or OH, and their acid addition salts,

characterised in that
a) a compound of formula III

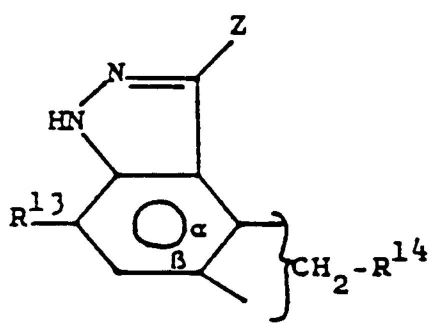

(III),

wherein Z has the meanings given above, $R^{14}$ represents $CH_2-NR^1R^2$ ($R^1$ and $R^2$ having the above meanings), CN or $CH_2-NH-COCF_3$, and $R^{13}$ is $NO_2$, $NH_2$ or $OCH_3$, it being possible for the group $CH_2-R^{14}$ to be in the $\alpha$- or $\beta$-position,

a1) if $R^{13}$ is $NO_2$, is reduced to the amino group,

a2) if $R^{13}$ is $NH_2$, is hydrolysed to the hydroxy group, or

a3) the amino group is reacted with a carboxylic acid chloride $R^3COCl$, a carboxylic acid anhydride $(R^3-CO)_2O$, wherein $R^3$ has the meanings given above, or with methanesulphonyl chloride, there being obtained compounds of formula I wherein X is $R^3CONH$ or $CH_3SO_2NH$,

a4) if $R^{13}$ is $OCH_3$, is subjected to an ether cleavage, there being obtained compounds of formula I wherein X is the hydroxy group and, if $R^{14}$ represents the grouping $CH_2-NH-COCF_3$, $R^1$ and $R^2$ are hydrogen;

b) a substituted phenylethylamine of formula IV

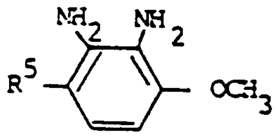 (IV),

wherein $R^5$ is hydrogen or the radical

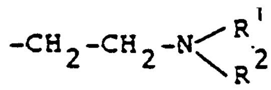 ,

with $R^1$ and $R^2$ having the above-indicated meanings, is reacted with a reagent R

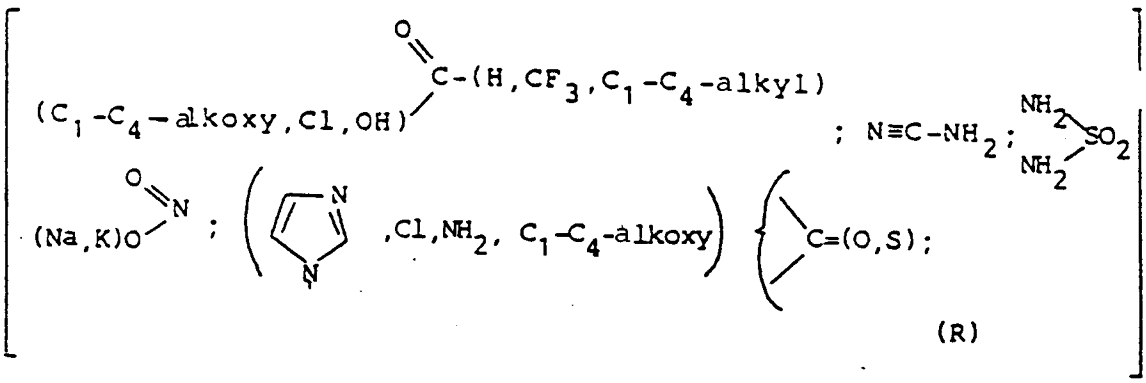

(R)

and, where appropriate after the introduction of $C_1$-$C_5$-alkyl groups on the N-atom of the aminoethyl side chain or, when $R^5$ is H, after the introduction of a formyl group ($R^5$ = CHO), conversion into a nitrovinyl group ($R^5$ = $CH=CH-NO_2$), reduction to an aminoethyl group ($R^5$ = $CH_2$-$CH_2$-$NH_2$) and $C_1$-$C_5$-alkylation at that $CH_2$-$CH_2$-$NH_2$ group, is subjected to ether cleavage.

**5.** A medicament consisting of one or more compounds according to claim 1 together with conventional auxiliary agents and excipients.

## Revendications

**1.** Dérivés de dopamine répondant à la formule générale I

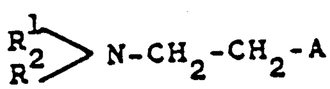 (I),

dans laquelle
A représente un radical phényle substitué de la structure

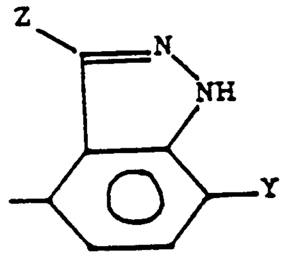

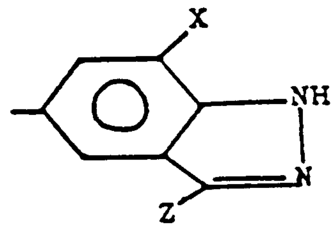

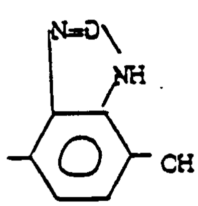

ou

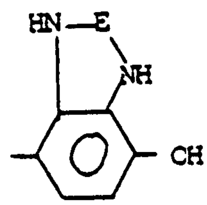

$R^1$ et $R^2$ sont identiques ou différents, et représentent de l'hydrogène, un alkyle en $C_{1-5}$ ou un allyle,
D est égal à

$$>C-R_4 \text{ ou } >N,$$

$R_4$ est de l'hydrogène, un alkyle en $C_1$-$C_4$, $CF_3$ ou $NH_2$,
E représente

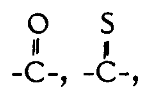

$-SO_2-$,
X représente OH, $NH_2$,

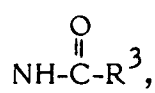

ou $NH-SO_2-CF_3$,
Y représente OH, $NH_2$,

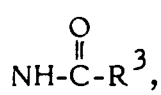

$NH-SO_2-CF_3$ ou $NH-SO_2-CH_3$,
$R^3$ représente un alkyle en $C_{1-4}$,
et Z représente H ou OH, et dans le cas où Z représente le groupe hydroxy, le radical A peut également se présenter dans la forme de base tautomère,
et leurs sels d'addition d'acide.

**2.** Bromhydrate de 7-hydroxy-4-[2-(N,N-di-propylamino)-éthyl]-2,3-dihydro-2-benzimidazolone.

**3.** N,N-Dipropyl-N-[2-(7-amino-1H-indazol-4-yl)-éthyl]-amine et son dichlorhydrate.

**4.** Procédé de préparation de dérivés de dopamine de la formule I

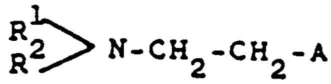 (I),

dans laquelle
A représente un radical phényle substitué de la structure

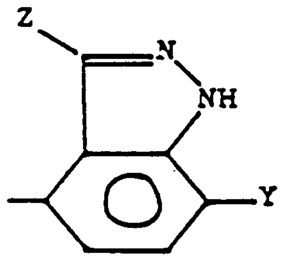

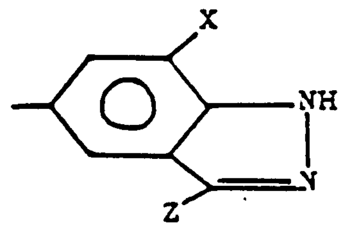,

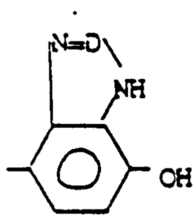

ou

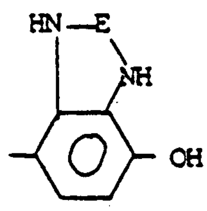

$R^1$ et $R^2$ sont identiques ou différents et représentent de l'hydrogène, un alkyle en $C_{1-5}$ ou un allyle,
D représente

$$\geqq C-R_4 \text{ ou } \geqq N$$

$R_4$ représente de l'hydrogène, un alkyle en $C_1$-$C_4$, du $CF_3$, du $NH_2$,
E représente

$$-\overset{\overset{O}{\|}}{C}-,\ -\overset{\overset{S}{\|}}{C}-,$$

$-SO_2-$,
X représente OH, $NH_2$,

$$NH-\overset{\overset{O}{\|}}{C}-R^3,$$

ou $NH-SO_2-CF_3$,
Y représente OH, $NH_2$,

$$NH-\overset{\overset{O}{\|}}{C}-R^3,$$

$NH-SO_2-CF_3$ ou $NH-SO_2-CH_3$,
$R^3$ est un alkyle en $C_{1-4}$, et

Z représente H ou OH, et de leurs sels d'addition d'acide, caractérisé en ce que

a) un composé de la formule III

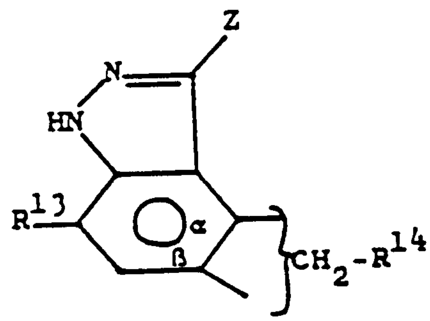

(III),

dans laquelle Z a la même signification que celle donnée précédemment, $R^{14}$ représente -$CH_2$-$NR^1R^2$ ($R^1$ et $R^2$ ont la même signification que celle donnée précédemment), CN ou $CH_2$-NH-$COCF_3$ et $R^{13}$ est identique à $NO_2$, $NH_2$ ou $OCH_3$, le groupe $CH_2$-$R^{14}$ pouvant se trouver en position soit $\alpha$ soit $\beta$,

a1) on le réduit en un groupe amino, dans le cas où $R^{13}$ est identique à $NO_2$,

a2) on l'hydrolyse en un groupe hydroxy dans le cas où $R^{13}$ est identique à $NH_2$, ou

a3) on fait réagir le groupe amino avec un chlorure d'acide carboxylique $R^3COCl$, un anhydride carboxylique $(R^3\text{-}CO)_2O$, où $R^3$ a la même signification que celle indiquée précédemment, ou avec du chlorure d'acide méthanesulfonique, des composés de la formule I, dans lesquels X a la signification de $R^3CONH$ ou $CH_3SO_2NH$, étant obtenus,

a4) on le soumet à une séparation d'éther dans le cas où $R^{13}$ est identique à $OCH_3$, des composés de la formule I, dans lesquels X représente le groupe hydroxy, étant obtenus, et, dans le cas où $R^{14}$ représente le groupement $CH_2$-NH-$COCF_3$, $R^1$ et $R^2$ sont de l'hydrogène,

b) on fait réagir une phényléthylamine substituée de la formule IV

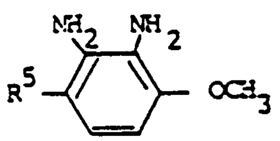

(IV),

dans laquelle $R^5$ est de l'hydrogène ou le radical

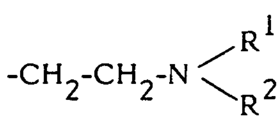

où $R^1$ et $R^2$ ont les significations déjà indiquées, avec un réactif R

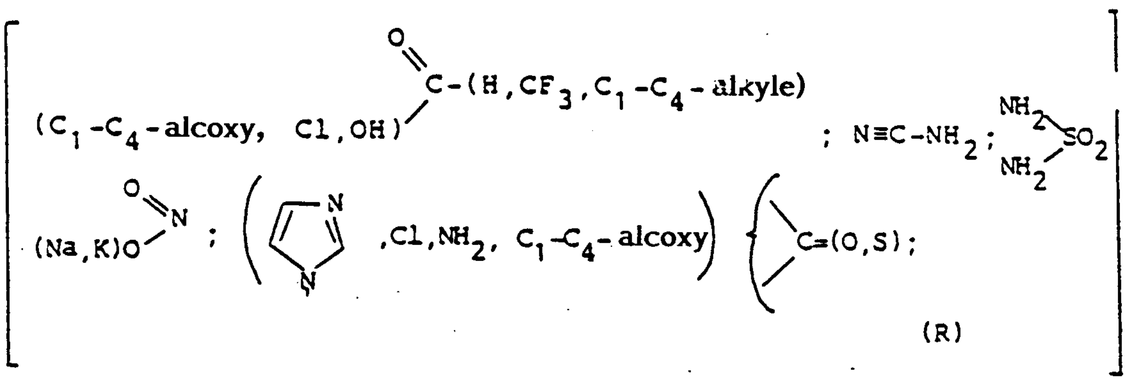

et éventuellement après introduction de groupes alkyle en $C_1$-$C_5$ sur l'atome N de la chaîne latérale aminoéthyle ou, dans le cas où $R^5$ = H, après introduction d'un groupe formyle ($R^5$ = CHO), transformation en un groupe nitrovinyle ($R^5 = CH = CH\text{-}NO_2$),réduction en un groupe aminoéthyle ($R^5 = CH_2\text{-}CH_2\text{-}NH_2$) et alkylation en $C_1$-$C_5$ sur ce groupe $CH_2\text{-}CH_2\text{-}NH_2$, on soumet à une séparation d'éther.

**5.** Médicaments constitués d'un ou de plusieurs composés suivant la revendication 1 et des adjuvants et supports courants.